# EUROPEAN PATENT APPLICATION

(11) **EP 4 810 021 A1**
(43) Date of publication of application: **23.09.2026**
(21) Application number: 24891764.3
(22) Date of filing: 13.11.2024
(51) Int. Cl.: A61K 9/16, A61K 38/08, A61P 5/24

(54) **SUSTAINED RELEASE FORMULATION WITH INCREASED INITIAL RELEASE AMOUNT AND MANUFACTURING METHOD THEREFOR**

(30) Priority: 16.11.2023 KR 20230159452
(71) Applicant: Peptron, Inc., Daejeon 34054 (KR)
(72) Inventor: CHOI, Ho Il, Daejeon 34054 (KR); CHO, Jae Pyoung, Cheongju-si Chungcheongbuk-do 28161 (KR); LEE, Ji Hyang, Cheongju-si Chungcheongbuk-do 28161 (KR)
(74) Representative: Potter Clarkson
(86) International application number: PCT/KR2024/017916
(87) International publication number: WO 2025/105818

(57) **Abstract**

A sustained-release formulation with an increased initial burstaccording to the present disclosure exhibits a rapid release within 24 hours after administration and can exhibit a continuous sustained-release effect thereafter, thereby achieving long-term drug efficacy persistence and an initial rapid release purpose at the same time. The sustained-release formulation comprising microspheres containing leuprorelin according to the present disclosure was found to have excellent bioavailability and be safe in the human body, and thus can be effectively and safely used for the treatment of various luteinizing hormone-releasing hormone-related diseases such as endometriosis, uterine myoma, prostate cancer, pre-menopausal breast cancer, and central precocious puberty.

## Description

### [Technical Field]

The present disclosure relates to a sustained-release formulation of leuprorelin having immediate release and sustained-release properties with an increased initial burst, and a manufacturing method therefor.

### [Background Art]

Luteinizing hormone releasing hormone (LHRH), also known as gonadotropin-releasing hormone (GnRH), is a hypothalamic decapeptide (pGlu-His-Trp-Ser-Tyr-Gly-Leu-Arg-Pro-Gly-NH2) that regulates the reproductive system of vertebrates. LHRH agonists deplete LHRH receptors in the pituitary gland to induce desensitization of pituitary receptors to LHRH stimulation, thereby inhibiting LH secretion. In addition, LHRH agonists and antagonists have been shown to be effective in the treatment of endometriosis, fibroma, polycystic ovary syndrome, breast cancer, ovarian cancer, and endometrial cancer, and gonadotropic pituitary desensitization during medical-assisted childbirth protocols in women, the treatment of benign prostatic hyperplasia, polymorphism, and prostate cancer in men, and the treatment of precocious puberty in men or women.

Leuprorelin acetate, a representative LHRH agonist, has the characteristics of rapidly decreasing blood concentration after administration and disappearing within a few hours due to a short half-life during subcutaneous or intramuscular injection. As a result, despite being administered as an injection, there was the inconvenience of having to be administered daily to maintain drug efficacy, causing discomfort to patients.

As such, as a method for minimizing the inconvenience caused by frequent administration and increasing drug compliance of patients, research was conducted to sustain drug release for a long time period by applying drugs to an injectable drug delivery system. Particularly, sustained-release formulations have been developed in the form of microspheres prepared using biodegradable polymers.

However, although leuprorelin acetate requires sufficient drug release at the early administration to exhibit a pharmacological effect, these sustained-release formulations only achieve the goal of a long-term delayed release, but have a disadvantage of failing to release the drug at a high initial concentration.

As such, conventional developed leuprorelin sustained-release formulations have limitations of exhibiting no sufficient pharmacological effect, and there is a need for a novel formulation that can achieve long-term drug efficacy persistence and an initial rapid release purpose at the same time.

### [Disclosure]

### [Technical Problem]

The present inventors confirmed that effective initial release and sustained-release effects could be simultaneously achieved when a trace amount of leuprorelin or a salt thereof was added to the outside of sustained-release microspheres encapsulated with the leuprorelin or the salt thereof while researching a novel formulation that overcome the initial release delay of sustained-release leuprorelin microspheres and simultaneously exhibited a continuous sustained-release thereafter, and then completed the present disclosure.

Therefore, an object of the present disclosure is to provide a leuprorelin sustained-release formulation with an increased initial burst and a manufacturing method therefor.

### [Technical Solution]

In order to achieve the object, an aspect of the present disclosure provides a manufacturing method of a sustained-release formulation with an increased initial burst, including: 1) preparing a spray solution by dissolving leuprorelin or a salt thereof and a biodegradable polymer in an organic solvent; 2) preparing microspheres encapsulated with the leuprorelin or the salt thereof by spray-drying the spray solution; and 3) preparing microspheres from which a residual solvent is removed by dispersing and washing the microspheres in an aqueous polyvinyl alcohol solution; and further including adding a freeze-dried powder of the leuprorelin or the salt thereof to the outside of the microspheres.

Another aspect of the present disclosure provides a sustained-release formulation with an increased initial burst, prepared by the above-described manufacturing method.

Yet another aspect of the present disclosure provides a sustained-release formulation with an increased initial burst, including sustained-release microspheres containing leuprorelin or a salt thereof and a biodegradable polymer and encapsulated with the leuprorelin or the salt thereof; and leuprorelin or a salt thereof outside the sustained-release microspheres.

### [Advantageous Effects]

According to the present disclosure, a sustained-release formulation with an increased initial burst release exhibits a rapid release within 24 hours after administration and can exhibit a continuous sustained-release effect thereafter, thereby achieving long-term drug efficacy persistence and an initial rapid release purpose at the same time. The sustained-release formulation including microspheres containing leuprorelin according to the present disclosure was found to have excellent bioavailability and be safe in the human body, and thus can be effectively and safely used for the treatment of various luteinizing hormone-releasing hormone-related diseases such as endometriosis, uterine myoma, prostate cancer, pre-menopausal breast cancer, and central precocious puberty.

### [Description of Drawings]

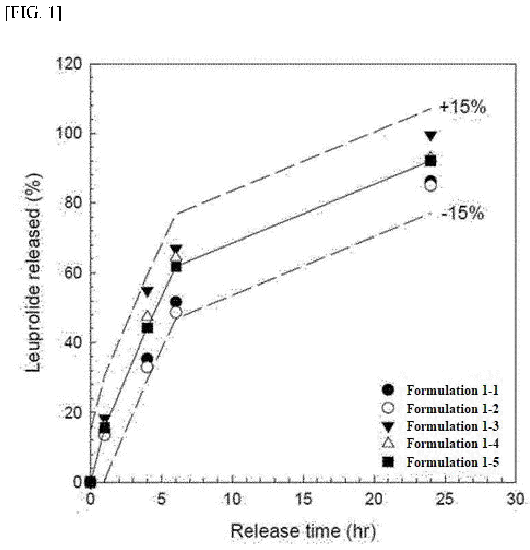

FIG. 1 is a diagram showing results of confirming a difference in release rate according to a difference in inherent viscosity of poly(lactide-co-glycolide) (PLGA) used for the preparation of microspheres.

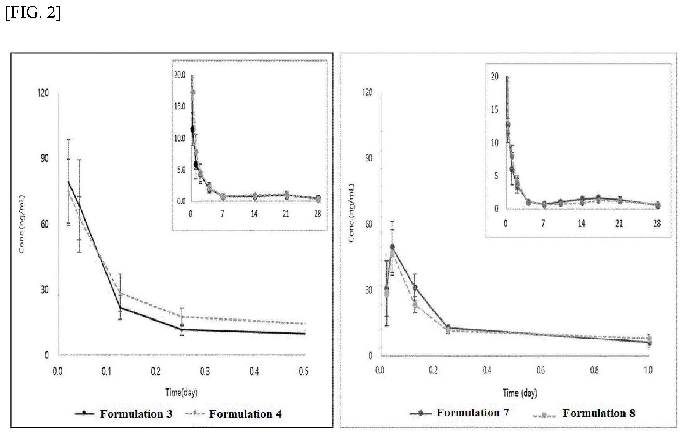

FIG. 2 is a diagram showing drug concentrations in serum over time for 0.5 or 1 day and drug concentrations in serum for 28 days (inset diagram) for Formulations 3 and 4, which contain a small amount of leuprorelin acetate (immediate release) outside microspheres, manufactured by the manufacturing method of the present disclosure, and Formulations 7 and 8, which do not contain immediate-release leuprorelin as Comparative Examples.

### [Best Mode]

The present disclosure relates to a sustained-release formulation with an increased initial burst that simultaneously exhibits immediate-release and sustained-release characteristics, and a manufacturing method therefor.

The sustained-release formulation with the increased initial burst of the present disclosure has an advantage of simultaneously improving drug compliance of patients and a drug effect by inducing a rapid release of a leuprorelin drug with an important initial release within 24 hours and then continuously releasing the drug in a sustained manner.

The present disclosure provides a manufacturing method of a sustained-release formulation with an increased initial burst, including: 1) preparing a spray solution by dissolving leuprorelin or a salt thereof and a biodegradable polymer in an organic solvent; 2) preparing microspheres encapsulated with the leuprorelin or the salt thereof by spray-drying the spray solution; and 3) preparing microspheres from which a residual solvent is removed by dispersing and washing the microspheres in an aqueous polyvinyl alcohol solution; and further including adding a freeze-dried powder of the leuprorelin or the salt thereof to the outside of the microspheres.

The leuprorelin or the salt thereof which is a pharmacologically active ingredient of the present disclosure may be characterized by being included in microspheres as the sustained-release formulation and simultaneously further included outside the microspheres to achieve an initial rapid release.

In the present disclosure, step 1) is a step of preparing the spray solution by dissolving the leuprorelin or the salt thereof and the biodegradable polymer in the organic solvent.

The leuprorelin may be used interchangeably with leuprolide and may be represented by the following Chemical Formula 1.

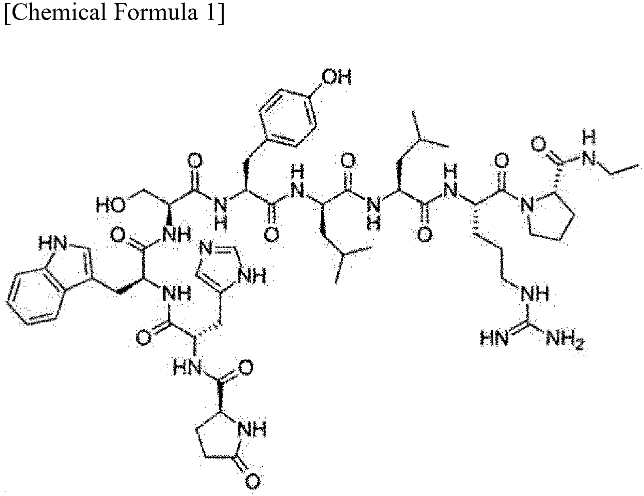

In the present disclosure, the salt of the leuprorelin may include all pharmaceutically acceptable salts without limitation, and may include, for example, acid addition salts or quaternary ammonium salts. The acid addition salts may include all acid addition salts formed from free acids, and for example, may be free acid-derived addition salts including organic acids such as citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid, and aspartic acid, and inorganic acids such as hydrochloric acid, bromic acid, sulfuric acid, and phosphoric acid. Most preferably, the salt of the leuprorelin may be leuprorelin acetate.

In the present disclosure, the biodegradable polymer is a polymer that is harmless to the human body by slowly decomposing when administered into the body, and may be at least one selected from the group consisting of polylactide, polyglycolide, polylactide-co-glycolide, polyorthoester, polyanhydride, polyhydroxybutyrate, polycaprolactone, and polyalkyl carbonate. Preferably, the polylactide-co-glycolide may be used, and when preparing sustained-release microspheres using the method of the present disclosure, PLGA having an inherent viscosity of 0.10 to 1.0 dL/g, preferably 0.14 to 0.22 dL/g may be used. The PLGA may have a molar ratio of lactide to glycolide of 50 to 100:50 to 0, 60 to 90:40 to 10, or 70 to 80:30 to 20, and preferably 75:25. For example, when preparing the sustained-release microspheres of the present disclosure, PLGA having the molar ratio of lactide to glycolide of 75:25 and the inherent viscosity of 0.14 to 0.22 dL/g may be used.

Since it has been confirmed that a release (elution) rate may be maintained equally even when the inherent viscosity is varied to 0.14 to 0.22 dL/g, polylactide-co-glycolide having the inherent viscosity of 0.14 to 0.22 dL/g may be used.

The biodegradable polymer may be included in an amount of 70 to 95 wt%, more preferably 80 to 95 wt%, and most preferably 85 to 95 wt% based on the total weight of the microspheres.

The organic solvent used may be selected without limitation as any solvent capable of dissolving the biodegradable polymer and the leuprorelin or the salt thereof, and may be selected according to a type of biodegradable polymer, and Class 3 and 4 solvents specified in the ICH guidelines may be selected to use low-toxicity solvents.

In an embodiment of the present disclosure, glacial acetic acid, i.e., acetic acid, was selected and used as the organic solvent so as to provide a formulation with high safety compared to using methylene chloride of Class 2 as a solvent. Accordingly, the organic solvent of the present disclosure may be acetic acid or glacial acetic acid, and preferably glacial acetic acid. The glacial acetic acid refers to acetic acid having high purity of 99.5% or higher.

In step 1) of the present disclosure, the spray solution is prepared by dissolving the leuprorelin or the salt thereof and the biodegradable polymer in the organic solvent.

The microspheres containing the leuprorelin or the salt thereof may be prepared by a method for manufacturing sustained-release injections known in the art. Various methods such as coacervation, melt extrusion, spray drying, solvent extraction, and solvent evaporation methods [double-emulsion evaporation (W/O/W; water/oil/water) and single-emulsion evaporation (O/W; oil/water)] have been known. Among these, like a phase separation method, the preparation of the microspheres by the single or double-emulsion evaporation method has limitations of difficulty in removing organic solvents used for dissolving biodegradable polymers, process difficulty due to changes in solvent removal rate during mass production, allergic reactions caused by gelatin used to increase the viscosity of a primary emulsion, possibility of drug denaturation and loss of activity due to strong shear forces applied to prepare small microspheres during primary emulsion preparation, and drug encapsulation rate.

Therefore, in the present disclosure, the microspheres encapsulated with the drug may be prepared preferably by a spray drying method among methods for manufacturing sustained-release injections known in the art, and step 2) of the present disclosure is a step of preparing microspheres encapsulated with the leuprorelin or the salt thereof by spray-drying the spray solution.

The spray drying of the present disclosure is performed by evaporating the solvent with hot air while supplying and spraying the spray solution prepared in step 1) to a nozzle. Appropriate supply rate, temperature, time, and supply amount may be controlled depending on a type of biodegradable polymer used, the content in microspheres, the content of leuprorelin or a salt thereof, and the content of a solvent. More preferably, the spray drying may be performed using a spray dryer equipped with an ultrasonic nozzle, and the spray solution may be sprayed into a drying chamber through the ultrasonic nozzle at the top of a sprayer while conveying the spray solution at a flow rate of 1 to 10 ml per minute. The temperature during spray drying may be 100 to 150°C, 120 to 145°C, preferably 130 to 140°C, and the microspheres encapsulated with the leuprorelin or the salt thereof may be prepared through the spray drying step as described above.

The spray drying step of the present disclosure may be performed by adjusting a nozzle frequency according to a desired microsphere size, and preferably, may be performed using an ultrasonic nozzle having a nozzle frequency of 40 to 80 kHz or 50 to 70 kHz. By using the ultrasonic nozzle having the nozzle frequency described above, a sustained-release formulation with an increased initial burst and an average particle size of 15 to 25 µm may be manufactured.

The average particle size is generally a smaller particle size than sustained-release microspheres suitable for 23-25G needles, and the sustained-release formulation may be subcutaneously injected using a 26G needle, and thus has an advantage of improving drug compliance of patients.

Step 3) of the present disclosure is a step of preparing microspheres from which a residual solvent has been removed by dispersing and washing the prepared microspheres in an aqueous polyvinyl alcohol solution. Through the dispersing process of step 3) above, the residual solvent remaining in the prepared microspheres immediately after spray drying may be removed, and at the same time, the microspheres may be dispersed more effectively in the injection.

At this time, the aqueous polyvinyl alcohol solution may be used at a concentration of 0.05 to 2.0 (w/v)%, preferably 0.05 to 1.0 (w/v)% or 0.1 to 0.5 (w/v)%, and more preferably 0.05 to 0.2 (w/v)%.

The molecular weight of the polyvinyl alcohol used may be 3,000 to 300,000 Da, and more preferably 5,000 to 100,000 Da, but is not limited thereto.

The dispersing step of the present disclosure may be performed by stirring the aqueous polyvinyl alcohol solution and the microspheres using a stirrer, and at this time, the stirring may be performed at room temperature at 1,000 to 3,000 rpm for 1 minute to 5 hours, preferably 5 minutes to 3 hours, and more preferably 10 minutes to 2 hours.

Through the dispersing and washing step described above, the residual solvent may be removed and microspheres with improved injectability may be prepared, and preferably, a final formulation may be produced with a residual amount of 0.5% (w/w) or 5,000 ppm or less, which is the control level recommended by the ICH Q3C (R8) guideline. More preferably, the sustained-release formulation of the present disclosure manufactured by performing the dispersing and washing step may be characterized by removing at least 99%, more preferably at least 99.5% of the residual solvent, but is not limited thereto.

In addition, the present disclosure provides a manufacturing method characterized by further including a step of adding a freeze-dried powder of leuprorelin or a salt thereof to the outside of the microspheres.

The step is a step for effectively increasing the initial burst of the leuprorelin or the salt thereof, and the leuprorelin or the salt thereof may be added to the outside of the microspheres encapsulated with the leuprorelin or the salt thereof prepared through steps 1) to 3), so that the leuprorelin or the salt thereof included on the outside may be released immediately in the early stage.

More specifically, the adding may include 4-1) preparing microspheres by suspending the microspheres prepared in step 3) in water for injection and water-soluble polyalcohol and then freeze-drying and crushing the suspension; and 4-2) filling the microspheres of step 4-1) with the freeze-dried powder of the leuprorelin or the salt thereof.

Alternatively, the adding may include suspending the microspheres prepared in step 3) in water for injection in which the leuprorelin or the salt thereof and the water-soluble polyalcohol are dissolved, and then freeze-drying and crushing the suspension.

That is, the process of adding the leuprorelin or the salt thereof to the outside of the microspheres encapsulated with the leuprorelin or the salt thereof is adding a crushed product or powder of the freeze-dried leuprorelin or salt thereof. A process of separately freeze-drying and crushing the leuprorelin or the salt thereof and then filling the microspheres with the leuprorelin or the salt thereof, or resuspending the microspheres and the leuprorelin or the salt thereof in water for injection and then freeze-drying, crushing, and adding the microspheres and the leuprorelin or the salt thereof together, may be used without limitation.

At this time, water-soluble polyalcohol may be used for the freeze-drying process, and the water-soluble polyalcohol may be mannitol, sorbitol, or dulcitol.

Leuprorelin or a salt thereof for immediate release to be added to the outside of the microspheres may be added in an amount of 2 to 8% (w/w), preferably 4 to 6% (w/w), with respect to the total weight of an active ingredient, that is, the leuprorelin or the salt thereof (sustained-release) included in the sustained-release microspheres. When the range is exceeded, not only an excessively immediate release may be induced, but persistence is also reduced, making it difficult to achieve drug efficacy for a desired period, and when less than the range is added, it is difficult to achieve a rapid increase in blood drug concentration within the initial 24 hours. In a preferred embodiment, among total active ingredients, 92 to 98% (w/w) of the leuprorelin or the salt thereof is provided in a form encapsulated in the microspheres, and 2 to 8% (w/w) of the leuprorelin or the salt thereof may be provided in a separate freeze-dried powder form or additionally encapsulated form outside the microspheres.

Through the manufacturing method, a sustained-release formulation with improved bioavailability and an increased initial burst may be manufactured, which may be characterized by a high initial elution rate within 24 hours and a drug duration of about 30 to 65 days. In a preferred embodiment, the initial release within 24 hours in vitro or in vivo may be 10% or less, 8% or less, or 6% or less.

In the present disclosure, 'the increased initial burst (initial release)' means a sustained-release formulation that exhibits an increased Cₘₐₓ value of about 30 to 50% or more and a high initial burst within 24 hours compared to sustained-release leuprorelin microspheres in which leuprorelin or a salt thereof is not added to the outside of the microspheres.

Without impairing the objects and effects of the present disclosure, in addition to the processes of steps 1) to 4) above, additional processes necessary for preparing and recovering the microspheres encapsulated with the leuprorelin or the salt thereof of the present disclosure, such as redispersing, mixing, recovering, stirring, heating, and washing steps, may be performed by processes known to those skilled in the art.

The present disclosure provides a sustained-release formulation with an increased initial burst, including sustained-release microspheres containing leuprorelin or a salt thereof and a biodegradable polymer and encapsulated with the leuprorelin or the salt thereof; and leuprorelin or a salt thereof outside the sustained-release microspheres.

The sustained-release formulation with the increased initial burst may be prepared by the manufacturing method of the sustained-release formulation with the increased initial burst described above.

The leuprorelin or the salt thereof included outside the sustained-release microspheres may be included in an amount of 2 to 8% (w/w), preferably 4 to 6% (w/w) of the total weight of the active ingredients.

In the sustained-release formulation, the leuprorelin or the salt thereof may be present in two forms, including a form encapsulated in sustained-release microspheres and a form existing outside the microspheres, thereby achieving initial rapid release and sustained- release at the same time.

The sustained-release formulation of the present disclosure is preferably an injection, and particularly preferably may be provided in the form of a prefilled syringe. When provided in the form of the prefilled syringe, convenience during the suspension preparation process is increased, and a risk of contamination may be reduced by minimizing an external contact.

When the sustained-release formulation of the present disclosure is an injection, the sustained-release formulation may be administered via various routes such as subcutaneous, intradermal, intravenous, intramuscular, or intraperitoneal routes.

The sustained-release formulation including the microspheres containing the leuprorelin or the salt thereof according to the present disclosure was found to have excellent bioavailability and be safe in the human body. Since 2 to 8% (w/w) of the leuprorelin or the salt thereof is included outside the microspheres with respect to the total weight of the active ingredients and an effective *in vivo*-profile is exhibited, the sustained-release formulation can be used alone or in combination with other known treatment methods for the treatment of various luteinizing hormone-releasing hormone-related diseases such as endometriosis, uterine myoma, prostate cancer, pre-menopausal breast cancer, and central precocious puberty.

The numerical values described herein should be interpreted to include an equivalent range unless otherwise specified.

Hereinafter, preferred Examples will be proposed in order to help in understanding of the present disclosure. However, the following Examples are just provided to more easily understand the present disclosure and the contents of the present disclosure are not limited by Examples.

### [Modes]

### Example 1 - Method for preparing immediate/sustained-release formulation of leuprorelin acetate

### 1.1 Confirmation of release effect due to difference in PLGA inherent viscosity

In the present disclosure, PLGA (Evonik Co., Ltd., Resomer^{®}752H, inherent viscosity 0.14 to 0.22 dL/g, hereinafter RG752H) was used as a biodegradable polymer. Since the inherent viscosity of RG752H was controlled to be in the range of 0.14 to 0.22 dL/g, an experiment was conducted to determine whether a drug release effect varied depending on the inherent viscosity of the biodegradable polymer used. Microspheres were prepared by using leuprorelin acetate ((PPL, Sweden) (Formulation 1-1) or (ANYGEN Co., Ltd., South Korea) (Formulations 1-2 to 1-5)) and D,L-lactic acid and varying the inherent viscosity of PLGA of 0.16 dL/g (Formulation 1-5), 0.18 dL/g (Formulation 1-4), 0.19 dL/g (Formulation 1-3), and 0.20 dL/g (Formulations 1-2 and 1-1). The ingredients were dissolved in glacial acetic acid to prepare a spray solution, and the prepared spray solution was sprayed into a drying chamber through an ultrasonic nozzle and then spray-dried at 135 ± 5°C to prepare PLGA microspheres encapsulated with a main ingredient. To remove a residual solvent in the microspheres after spray-drying, the prepared PLGA microspheres were dispersed in a 0.1 (w/v)% polyvinyl alcohol (PVA) solution, and washed and filtered with water for injection to remove the residual PVA and glacial acetic acid.

The elution patterns were evaluated according to an inherent viscosity used in the prepared formulations. An accelerated release test was conducted according to test conditions below, and samples collected during the release test were analyzed using HPLC, and the results were illustrated in FIG. 1.
- Release tester: Rotary type release device (Equipment number: LDT-PT-008)
- Release temperature: 50°C
- Number of rotations: 20 rpm
- Sampling: 1 hr, 4 hrs, 6 hrs, 24 hrs
- Release solution: 10 mM lactic acid buffer, pH 3.2 (0.4% PVA + 0.1% Tween 80)

At all release (elution) rate comparison time points, if a difference in the average release (elution) rate between the reference formulation and the comparison formulation was within ± 15%, and if a similarity factor (f2) was 50 or higher when setting all release (elution) rate measurement times as comparison time points, all of the release (elution) rates were judged to be equivalent. The similarity factor (f2) was calculated using the following Equation.${f}_{2} = 50 ⋅ log \left({\left[1+\frac{1}{n}{\sum }_{t = 1}^{n} {\left({R}_{l}-{T}_{l}\right)}^{2}\right]}^{- 0.5}\times 100\right)$
- n: Number of time points for measuring release (elution) (%)
- Rt: Release (elution) rate (%) of reference formulation
- Tt: Release (elution) rate (%) of comparative formulation

As illustrated in FIG. 1, when other formulations were compared with microspheres (Formulation 1-5) prepared using RG752H with the lowest inherent viscosity of 0.16 dL/g as a reference formulation, it was confirmed that differences therebetween were within 15% at all release comparison time points.

In addition, the similarity factor (f2) value had values of 51 to 82, and as a result, it was confirmed that the release patterns of all experimental groups were equivalent.

Additionally, for Formulations 1-1 to 1-5, the average particle size (span value), content, residual solvent (acetic acid), and moisture content were confirmed.

Formulations 1-1 to 1-5 prepared by varying RG752H inherent viscosity and experimental values measured in the corresponding formulations were shown in Table 1.

**[Table 1]**

| Classification | Immediate and sustained-release | RG752H inherent viscosity | PVA concentration in dispersion process solution | Residual solvent (acetic acid) | Average particle size (span value) | Drug and polymer content in microspheres |
|---|---|---|---|---|---|---|
| Formulation 1-1 | Sustained-release | 0.2 dL/g | PVA 0.1% | 0.32% | 16.3 µm (1.41) | Drug: 3.75 mg (10%) |
| | | | | | | Polymer: 33.75 mg (90%) |
| Formulation 1-2 | Sustained-release | 0.2 dL/g | PVA 0.1% | 0.32% | 15.6 µm (1.45) | Drug: 3.75 mg (10%) |
| | | | | | | Polymer: 33.75 mg (90%) |
| Formulation 1-3 | Sustained-release | 0.19 dL/g | PVA 0.1% | 0.26% | 16.9 µm (1.46) | Drug: 3.75 mg (10%) |
| | | | | | | Polymer: 33.75 mg (90%) |
| Formulation 1-4 | Sustained-release | 0.18 dL/g | PVA 0.1% | 0.27% | 15.8 µm (1.45) | Drug: 3.75 mg (10%) |
| | | | | | | Polymer: 33.75 mg (90%) |
| Formulation 1-5 | Sustained-release | 0.16 dL/g | PVA 0.1% | 0.25% | 16.2 µm (1.44) | Drug: 3.75 mg (10%) |
| | | | | | | Polymer: 33.75 mg (90%) |

As can be seen in Table 1, it was confirmed that there were no significant differences in residual solvent, average particle size, drug and polymer contents in microspheres, and residual solvent in all experimental groups.

The results indicated that if biodegradable polymers were used with the same composition, formulations with equivalent release patterns and quality may be manufactured even if their inherent viscosity varied up to 0.04 dL/g within the manufacturer's specification range. Therefore, in the following Preparation Examples of the present disclosure, RG752H with the inherent viscosity of 0.20 dL/g or 0.21 dL/g was used to prepare microspheres.

### 1.2 Preparation method of Preparation Examples 1 to 6

A spray solution was prepared by dissolving leuprorelin acetate (ANYGEN Co., Ltd., South Korea), D,L-lactic acid, and PLGA (RG752H, inherent viscosity 0.20 dL/g or 0.21 dL/g) in glacial acetic acid. At this time, the drug was loaded into the microspheres at approximately 10% (w/w).

More specifically, a spray solution was prepared by dissolving leuprorelin acetate and PLGA in glacial acetic acid at a weight ratio of about 9 to 10 times higher than the mixture thereof, and at this time, the leuprorelin acetate and the PLGA were dissolved in glacial acetic acid at a weight ratio of 1:9 to 1:9.5. The prepared spray solution was sprayed into a drying chamber through a 60 kHz ultrasonic nozzle and then spray-dried at 135 ± 5°C to prepare PLGA microspheres encapsulated with a main ingredient.

To remove a residual solvent in the microspheres after spray drying, the prepared PLGA microspheres were dispersed and filtered at 1,500 rpm for 20 minutes using 0.1 to 1.0 (w/v)% of a polyvinyl alcohol (PVA) solution in water for injection. Thereafter, the microspheres were washed and filtered again with water for injection to remove PVA and glacial acetic acid remaining in the microspheres. The mannitol solution and the washed microspheres were suspended in water for injection, and freeze-dried, and the dried microspheres were crushed through a 250 µm mesh sieve.

During the process of suspending the washed microspheres, leuprorelin acetate was additionally dissolved in the mannitol solution in an amount of 5% (w/w) or less with respect to the total active ingredient content (target dose), and then freeze-dried and crushed to prepare a final formulation in which leuprorelin acetate was filled outside the microspheres in addition to the active ingredient included in the sustained-release microspheres.

Alternatively, a final formulation was prepared in which leuprorelin acetate of 5% (w/w) or less of the total active ingredient content was filled outside the microspheres by adding leuprorelin acetate immediately after removing PVA and residual glacial acetic acid from the microspheres and performing freeze-drying and crushing together, or by adding and crushing separately the freeze-dried leuprorelin acetate to the microspheres after freeze-drying.

The microspheres were prepared by setting a particle size to 7.0 µm to 10.0 µm at D10, 18.0 µm to 23.5 µm at D50, and 33.5 µm to 45.5 µm at D90 so that the average particle size was 20 ± 3 µm.

### 1.3 Preparation method of Comparative Preparation Examples 1 and 2

A spray solution was prepared by dissolving leuprorelin acetate (PPL, Sweden), D,L-lactic acid, and PLGA (RG752H, inherent viscosity 0.21 dL/g) in glacial acetic acid. At this time, the drug was loaded into microspheres at 10% (w/w). The prepared spray solution was sprayed into a drying chamber through an ultrasonic nozzle and then spray-dried at 135 ± 5°C to prepare PLGA microspheres encapsulated with a main ingredient. To remove a residual solvent in the microspheres after spray drying, the prepared PLGA microspheres were dispersed in distilled water (Formulation 7, Comparative Preparation Example 1) or a 0.01 (w/v)% PVA solution (Formulation 8, Comparative Preparation Example 2), washed and filtered with water for injection to remove residual PVA and glacial acetic acid, and freeze-dried to prepare microspheres containing leuprorelin acetate as a final formulation.

Preparation Examples 1 to 6 (Formulations 1 to 6) and Comparative Preparation Examples 1 and 2 (Formulations 7 and 8) prepared by the method were shown in Table 2.

**[Table 2]**

| **Classification** | **Immediate and sustained-release** | **Main ingredient** | **RG752H inherent viscosity** | **PVA concentration in dispersion process solution** |
|---|---|---|---|---|
| Formulation 1 (Preparation Example 1) | Immediate and sustained-release | Interior 3.638 mg | 0.20 dL/g | PVA 0.1% |
| | | Exterior 0.180 mg | | |
| Formulation 2 (Preparation Example 2) | Immediate and sustained-release | Interior 3.675 mg | 0.21 dL/g | PVA 0.1% |
| | | Exterior 0.075 mg | | |
| Formulation 3 (Preparation Example 3) | Immediate and sustained-release | Interior 3.675 mg | 0.21 dL/g | PVA 1.0% |
| | | Exterior 0.075 mg | | |
| Formulation 4 (Preparation Example 4) | Immediate and sustained-release | Interior 3.675 mg | 0.21 dL/g | PVA 0.1% |
| | | Exterior 0.075 mg | | |
| Formulation 5 (Preparation Example 5) | Immediate and sustained-release | Interior 3.675 mg | 0.21 dL/g | PVA 0.1% |
| | | Exterior 0.176 mg | | |
| Formulation 6 (Preparation Example 6) | Immediate and sustained-release | Interior 3.675 mg | 0.21 dL/g | PVA 0.1% |
| | | Exterior 0.278 mg | | |
| Formulation 7 (Comparative Preparation Example 1) | Sustained-release | Interior 3.75 mg | 0.21 dL/g | DW |
| Formulation 8 (Comparative Preparation Example 2) | Sustained-release | Interior 3.75 mg | 0.21 dL/g | PVA 0.01% |

### Example 2. Measurement of particle size of PLGA microspheres encapsulated with leuprorelin acetate

The most important factor for determining the particle size of microspheres is a nozzle frequency. In general, a relationship between a droplet diameter and a frequency of a spray solution follows the following Lang formula (Equation 2).${d}_{h} = {0.73}^{3} \sqrt{\frac{T}{{{ρf}_{a}}^{2}}}$

(T = Surface tension, ρ= Solution density, fa = Nozzle frequency)

Based on the formula, it was predicted that the theoretical size of the microspheres according to the nozzle frequency would be 35.7 µm at 25 kHz, 19.9 µm at 60 kHz, and 12.5 µm at 120 kHz, and an experiment was performed by selecting a frequency of 60 kHz to prepare microspheres with a particle size of around 20 µm based on an average particle size.

Table 3 showed results of measuring the particle size of Formulation 1 (Preparation Example 1) among microspheres produced by PEPTRON Co., Ltd. using an ultrasonic nozzle with a frequency of 60 kHz.

**[Table 3]**

| no. | Diameter (µm) | | | | Span value |
|---|---|---|---|---|---|
| | D₁₀ | D₅₀ | D₉₀ | Mean | |
| Formulation 1 | 8.32 | 20.26 | 39.22 | 22.10 | 1.53 |
| | 8.31 | 20.17 | 38.85 | 21.96 | 1.51 |
| | 8.29 | 20.51 | 40.10 | 22.50 | 1.55 |

As shown in Table 3, it was confirmed that PLGA microspheres encapsulated with leuprorelin acetate produced by setting the nozzle frequency to 60 kHz had an average particle size in the low 20 µm range. The PLGA microspheres generally has a smaller particle size than sustained-release microspheres suitable for 23-25G needles, and has an advantage of being able to be injected subcutaneously using a 26G needle.

### Example 3. Removal of polyvinyl alcohol (PVA) used in dispersion process and measurement of residual amount

The manufacturing method and the sustained-release microsphere formulation of the present disclosure have the advantage of being safer than a manufacturing process using methylene chloride of Class 2 by using glacial acetic acid of Class 3, which is a low-toxic solvent. Furthermore, in order to minimize the amount of residual solvent remaining in a finished pharmaceutical product, the present disclosure includes a process of dispersing, stirring and filtering the PLGA microspheres encapsulated with the main ingredient in 0.1 to 1.0% (1,000 ppm to 10,000 ppm) of an aqueous PVA solution to obtain microspheres, and then washing and filtering the microspheres with water for injection (WFI). This process may be repeated several times as needed.

A residue measurement experiment was performed to confirm whether the organic solvent and PVA were present at sufficiently low residue levels through the manufacturing process described above. A test solution for measuring the residual amount of PVA was prepared by adding 0.5 N sodium hydroxide to a certain amount of sample to dissolve the sample, and then adding 1N hydrochloric acid to neutralize the sample. The HPLC analysis conditions were set with a solution of sodium nitrate dissolved in acetonitrile as a mobile phase, a TSKgel G2500SWXL column (7.8 * 300 mm, 5 µm), an RID detector, a column temperature of 37°C, and a flow rate of 1 mL/min.

Table 4 shows results of measuring the residual amount of PVA in Formulations 1 to 4 prepared by the method of the present disclosure while varying the PVA concentration of the dispersion process solution from 0.1 to 1.0%.

The amount of PVA remaining in the final formulation is approximately 10 ppm or less on average, and this result indicates that at least 99% of PVA which has been used in the manufacturing process is removed.

In addition, as can be seen in Table 4, it was confirmed that the organic solvent (residual solvent) remaining in the final formulation was removed by at least 99.5%. In particular, in the case of Formulation 2, the level of the residual solvent was measured to be undetectable.

This means that in Formulations 1 to 4 of the present disclosure, a residual amount is present at 0.5% (w/w) or 5,000 ppm or less, which is the control level recommended by the ICH Q3C (R8) guideline.

**[Table 4]**

| | **PVA concentration in dispersion process solution** | **PVA residue measurement result** | **Residual solvent (acetic acid)** |
|---|---|---|---|
| Formulation 1 | PVA 0.1% | 9.38 ± 0.24 ppm | 0.41% |
| Formulation 2 | PVA 0.1% | 9.19 ± 0.55 ppm | N/A |
| Formulation 3 | PVA 1.0% | 9.20 ± 0.45 ppm | 0.31% |
| Formulation 4 | PVA 0.1% | 9.38 ± 0.34 ppm | 0.42% |

Taken together with the results, compared to an example using a maximum daily dose of 0.119 mg in a topical sustained-release implant, the residual PVA amount of the immediate and sustained-release formulation of the present disclosure shows a significantly low value of approximately 10 ppm, and the residual solvent is present in an amount of less than 0.5%, and thus it can be seen that a safe formulation can be prepared without toxicity issues caused by the PVA or residual solvent remaining in the finished pharmaceutical product.

### Example 4. Confirmation of immediate and sustained-release effect

### 4-1. Initial release evaluation (in vivo)

Immediate and sustained-release formulations 3 and 4 prepared by the method of the present disclosure, and sustained-release formulations 7 and 8 without additionally containing leuprorelin acetate to the outside of the microspheres were administered to rats, and the initial release was confirmed. Twenty 10-week-old Sprague-Dawley specific pathogen-free (SPF) rats were randomly divided into experimental groups with n = 5 and then administered subcutaneously in a single dose of 3 mg/kg. Subsequently, the serum was obtained from each rat by jugular vein blood collection at 0.5, 1, 3, 6, and 24 hours, 2, 4, 7, 14, 21, and 28 days, and stored at -80°C, and then the AUC of each experimental group was measured by LC-MS/MS analysis. The results of confirming Cₘₐₓ, Tₘₐₓ, and AUC values were shown in Table 5, and the results of measuring serum concentrations over time were shown in FIG. 2.

**[Table 5]**

| | Formulation 3 | Formulation 4 | Formulation 7 | Formulation 8 |
|---|---|---|---|---|
| Cₘₐₓ (ng/mL) | 80.1 ± 20.1 | 76.5 ± 14.2 | 49.54 ± 11.63 | 47.02 ± 10.42 |
| Tₘₐₓ (hr) | 0.6 ± 0.2 | 0.6 ± 0.2 | 1.00 ± 0.00 | 1.00 ± 0.00 |
| AUC₇₋ₜ (ng*day/ml) | 16.2 ± 6.4 | 18.0 ± 3.8 | 25.03 ± 3.81 | 20.21 ± 1.99 |
| AUC₀₋ₜ (ng*day/ml) | 44.9 ± 11.5 | 51.8 ± 5.5 | 50.20 ± 5.13 | 45.95 ± 3.56 |
| AUC_{INF} (ng*day/ml) | 49.4 ± 13.2 | 55.1 ± 5.3 | 55.35 ± 5.74 | 56.72 ± 9.33 |

- AUC₇₋ₜ: AUC from day 7 to the last sampling time point
- AUC₀₋ₜ: AUC from day 0 to the last sampling time point
- AUC_{INF}: AUC from day 0 to time point where concentration becomes 0 (time point estimated by drawing a trend line)

As can be seen in Table 5 and FIG. 2, Formulations 3 and 4, which were immediate and sustained-release formulations containing 2% of the active ingredient outside the microspheres based on the active ingredient content in the microspheres, had Cₘₐₓ of about 35 to 40% higher than that of sustained-release formulations 7 and 8, and the initial release within 24 hours was improved. This result indicates that the immediate and sustained-release formulation of the present disclosure can effectively improve the low initial release rate of existing sustained-release formulations.

### 4-2. Evaluation of in vivo pharmacokinetics and safety

3.75 mg of leuprorelin acetate was administered subcutaneously in the abdomen to healthy postmenopausal women by a single subcutaneous injection of Formulations 1 and 2 of the present disclosure, and pharmacokinetic characteristics and safety were confirmed. A pharmacokinetic evaluation group for Formulation 1 consisted of 36 women and a pharmacokinetic evaluation group for Formulation 2 consisted of 38 women, and the pharmacokinetic blood collection time points were confirmed a total of 17 times at 0 (before administration), 0.5, 1, 1.5, 2, 4, 8, 12, 24, 72, 168, 240, 336, 504, 672, 840, and 1008 hr.

In particular, as confirmed in Example 4-1, since Cₘₐₓ was a parameter related to the initial burst, to determine the increased initial release and bioavailability according to the content (%) of leuprorelin acetate added to the outside, Cₘₐₓ was compared and analyzed using Formulations 1 and 2, which contained 4.8% or 2% of leuprorelin acetate with respect to the total leuprorelin acetate content outside the microspheres. The Cₘₐₓ and AUC values identified in Formulations 1 and 2 were shown in Table 6 below.

**[Table 6]**

| | **Formulation 1 (n = 36)** | **Formulation 2 (n = 38)** |
|---|---|---|
| Cₘₐₓ (ng/mL) | 8096.694 | 5712.325 |
| AUC₇₋ₜ (ng*day/ml) | 77663.320 | 77218.699 |
| AUCₜ (ng*day/ml) | 162948.819 | 142688.146 |

As can be seen in Table 6, Formulation 1, which contained 4.8% of the immediate-release active ingredient with respect to the total active ingredient content outside the microspheres, showed a Cₘₐₓ value of approximately 30% higher than that of Formulation 2, which contained 2% of the immediate-release active ingredient.

Through these results, it was confirmed that a formulation containing less than 8% (w/w), preferably 4 to 6% (w/w) of leuprorelin acetate with respect to the total active ingredient content outside the microspheres can induce an effective initial release and an increased Cₘₐₓ value in animals and humans. That is, by using the formulation of the present disclosure in which only less than 8% of leuprorelin acetate of the total active ingredients is contained outside the sustained-release microspheres, and the remaining leuprorelin acetate is encapsulated in the sustained-release microspheres, it is possible to manufacture an immediate and sustained-release microsphere formulation that overcomes initial delayed release and has excellent bioavailability to exhibit an effective therapeutic effect.

Additionally, an in vivo safety evaluation of the formulation of the present disclosure was performed simultaneously. As a result, no clinically significant special-interest adverse events and local adverse events were reported during the clinical trials, and no adverse events with clinically significant differences in frequency and pattern of occurrence were also identified between the administration groups in the clinical trials. Except for some cases collected as mild adverse events, no clinically significant changes were found in vital signs, clinical laboratory tests, physical examinations, 12-lead electrocardiograms, and BMD tests performed after administration of a clinical drug during the clinical trial period.

In conclusion, it was determined that there were no clinically significant differences between the administered groups in safety evaluations, including adverse events, local adverse events, concomitant drugs, vital signs, physical examinations, clinical laboratory tests, 12-lead electrocardiograms, and BMD tests.

## Claims

1. A manufacturing method of a sustained-release formulation with an increased initial burst, the manufacturing method comprising:
1) preparing a spray solution by dissolving leuprorelin or a salt thereof and a biodegradable polymer in an organic solvent;
2) preparing microspheres encapsulated with the leuprorelin or the salt thereof by spray-drying the spray solution; and
3) preparing microspheres from which a residual solvent is removed by dispersing and washing the microspheres in an aqueous polyvinyl alcohol solution; and
further comprising adding a freeze-dried powder of the leuprorelin or the salt thereof to the outside of the microspheres.

2. The manufacturing method of claim 1, wherein the adding comprises:
4-1) preparing microspheres by suspending the microspheres prepared in step 3) in water for injection and water-soluble polyalcohol and then freeze-drying and crushing the suspension; and
4-2) filling the microspheres of step 4-1) with the freeze-dried powder of the leuprorelin or the salt thereof.

3. The manufacturing method of claim 1, wherein the adding comprises suspending the microspheres prepared in step 3) in water for injection in which the leuprorelin or the salt thereof and the water-soluble polyalcohol are dissolved, followed by freeze-drying and crushing the suspension.

4. The manufacturing method of claim 1, wherein the leuprorelin or the salt thereof added to the outside is added in an amount of 2 to 8% (w/w) based on the total weight of active ingredients.

5. The manufacturing method of claim 1, wherein the organic solvent is glacial acetic acid or acetic acid.

6. The manufacturing method of claim 1, wherein in the sustained-release formulation, at least 99% of the residual solvent is removed.

7. The manufacturing method of claim 2, wherein the water-soluble polyalcohol is mannitol, sorbitol, or dulcitol.

8. The manufacturing method of claim 1, wherein the biodegradable polymer is at least one selected from the group consisting of polylactide, polyglycolide, polylactide-co-glycolide, polyorthoester, polyanhydride, polyhydroxybutyrate, polycaprolactone, and polyalkyl carbonate.

9. The manufacturing method of claim 1, wherein the sustained-release formulation with the increased initial burst has an average particle size of 15 to 25 µm.

10. A sustained-release formulation with an increased initial burstmanufactured by the manufacturing method of any one of claims 1 to 9.

11. A sustained-release formulation with an increased initial burst, the sustained-release formulation comprising:
sustained-release microspheres comprising leuprorelin or a salt thereof and a biodegradable polymer and encapsulated with the leuprorelin or the salt thereof; and
leuprorelin or a salt thereof outside the sustained-release microspheres.

12. The sustained-release formulation of claim 11, wherein the amount of the leuprorelin or the salt thereof included outside the sustained-release microspheres is 2 to 8% (w/w) based on the total weight of active ingredients.
